# EUROPEAN PATENT APPLICATION

(11) **EP 2 756 850 A1**
(43) Date of publication of application: **23.07.2014**
(21) Application number: 13151858.1
(22) Date of filing: 18.01.2013
(51) Int. Cl.: A61K 39/008, C07K 14/44, G01N 33/569

(54) **Diagnosis of Leishmania infection**

(71) Applicant: PHILIPPS-UNIVERSITÄT MARBURG, 35037 Marburg (DE)
(72) Inventor: Abass, Elfadil, 35039 Marburg (DE); Steinhoff, Ulrich, 35037 Marburg (DE); Lohoff, Michael, 35091 Cölbe (DE)
(74) Representative: Stumpf, Peter

(57) **Abstract**

Visceral leishmaniasis belongs to the most neglected diseases world-wide and particularly affects the poorest people in developing countries. The disease is caused by protozoan parasites of the genus *Leishmania,* which cause a series of clinical pictures. One object of the present invention, therefore, is to provide an improved diagnostic tool for the detection of infections with pathogens of the genus *Leishmania.* With present invention a novel polypeptide from *Leishmania donovani* is provided, which causes a *Leishmania-*specific immune reaction. The invention refers to the diagnosis of leishmaniasis, in particular to the diagnosis of visceral leishmaniasis by the use of said new polypeptide. The present disclosure also relates to a vector which carries a nucleotide sequence encoding a polypeptide according to the invention. Furthermore, the invention is directed to a method for the detection of an infection with pathogens of the genus *Leishmania.*

SEQUENCE LISTING

<110>  Philipps-Universität Marburg

<120>  Diagnosis of Leishmania infection

<130>  TM680

<140>  EP
<141>  2012-01-18

<160>  10

<170>  PatentIn version 3.5

<210>  1
<211>  294
<212>  PRT
<213>  Leishmania donovani

<400>  1

Glu Leu Ala Thr Glu Trp Glu Asp Ala Leu Arg Glu Arg Ala Leu Ala
1               5                   10                  15

Glu Arg Asp Glu Ala Ala Ala Ala Glu Leu Asp Ala Ala Ala Ser Thr
            20                  25                  30

Ser Gln Asn Ala Arg Glu Ser Ala Ser Glu Arg Leu Thr Ser Leu Glu
        35                  40                  45

Gln Leu Leu Arg Glu Ser Glu Glu Arg Ala Gly Glu Leu Ala Ser Gln
    50                  55                  60

Leu Glu Ser Thr Thr Ala Ala Lys Met Ser Ala Glu Gln Asp Arg Glu
65                  70                  75                  80

Asn Thr Arg Ala Thr Leu Glu Gln Gln Leu Arg Asp Ser Glu Glu Arg
                85                  90                  95

Ala Ala Glu Leu Ala Ser Gln Leu Glu Ala Thr Ala Ala Ala Lys Ser
            100                 105                 110

Ser Ala Glu Gln Asp Arg Glu Asn Thr Arg Ala Ala Leu Glu Gln Gln
        115                 120                 125

Leu Arg Asp Ser Glu Glu Arg Ala Ala Glu Leu Ala Ser Gln Leu Glu
    130                 135                 140

Ser Thr Thr Ala Ala Lys Thr Ser Ala Glu Gln Asp Arg Glu Asn Thr
145                 150                 155                 160

Arg Ala Thr Leu Glu Gln Gln Leu Arg Asp Ser Glu Glu Arg Ala Ala
                165                 170                 175

Glu Leu Ala Ser Gln Leu Glu Ser Thr Thr Ala Ala Lys Met Ser Ala
            180                 185                 190

Glu Gln Asp Arg Glu Asn Thr Arg Ala Ala Leu Glu Gln Gln Leu Leu
        195                 200                 205

Glu Ser Glu Glu Arg Ala Gly Glu Leu Ala Ser Gln Leu Glu Ser Thr
    210                 215                 220

Thr Ala Ala Lys Met Ser Ala Glu Gln Asp Arg Glu Asn Thr Arg Ala
225                 230                 235                 240

Ala Leu Glu Gln Gln Leu Arg Asp Ser Glu Glu Arg Ala Ala Glu Leu
                245                 250                 255

Ala Ser Gln Leu Glu Ser Thr Thr Ala Ala Lys Met Ser Ala Glu Gln
            260                 265                 270

Asp Arg Glu Asn Thr Arg Ala Thr Leu Glu Gln Gln Leu Arg Asp Ser
        275                 280                 285

Glu Glu Arg Ala Ala Glu
    290

<210>  2
<211>  883
<212>  DNA
<213>  Leishmania donovani

<400>  2
gagctcgcaa ccgagtggga ggacgcactc cgcgagcgtg cgcttgcaga gcgtgacgaa      60

gccgctgcag ccgaacttga tgccgcagcc tctacttccc aaaacgcacg tgaaagcgcc     120

tccgagcggc taaccagcct tgagcagctg cttcgcgaat ccgaggagcg cgctggggag     180

ctggcgagtc agctggagtc cactactgct gcgaagatgt cggcggagca ggaccgcgag     240

aacacgaggg ccacgctaga gcagcagctt cgtgactccg aggagcgcgc tgcggagctg     300

gcgagccagc tggaggccac tgctgctgcg aagtcgtcgg cggagcagga ccgcgagaac     360

acgagggccg cgttggagca gcagcttcgt gactccgagg agcgcgctgc ggagctggcg     420

agtcagctgg agtccactac tgctgcgaag acgtcggcgg agcaggaccg cgagaacacg     480

agggccacgc tagagcagca gcttcgtgac tccgaggagc gcgctgcgga gctggcgagt     540

cagctggagt ccactactgc tgcgaagatg tcagcggagc aggaccgcga gaacacgagg     600

gccgcgctag agcagcagct tctcgaatcc gaggagcgcg ctggggagct ggcgagtcag     660

ctggagtcca ctactgctgc gaagatgtca gcggagcagg accgcgagaa cacgagggcc     720

gcgctagagc agcagcttcg tgactccgag gagcgcgctg cggagctggc gagtcagctg     780

gagtccacta ctgctgcgaa gatgtcggcg gagcaggacc gcgagaacac gagggccacg     840

ctagagcagc agcttcgtga ctccgaggag cgcgctgcgg agc                       883

<210>  3

<211>  915
<212>  DNA
<213>  Leishmania donovani

<400>  3
catcaccatc accatcacgg atccatggag ctcgcaaccg agtgggagga cgcactccgc      60

gagcgtgcgc ttgcagagcg tgacgaagcc gctgcagccg aacttgatgc cgcagcctct      120

acttcccaaa acgcacgtga aagcgcctcc gagcggctaa ccagccttga gcagctgctt      180

cgcgaatccg aggagcgcgc tggggagctg gcgagtcagc tggagtccac tactgctgcg      240

aagatgtcgg cggagcagga ccgcgagaac acgagggcca cgctagagca gcagcttcgt      300

gactccgagg agcgcgctgc ggagctggcg agccagctgg aggccactgc tgctgcgaag      360

tcgtcggcgg agcaggaccg cgagaacacg agggccgcgt tggagcagca gcttcgtgac      420

tccgaggagc gcgctgcgga gctggcgagt cagctggagt ccactactgc tgcgaagacg      480

tcggcggagc aggaccgcga gaacacgagg gccacgctag agcagcagct tcgtgactcc      540

gaggagcgcg ctgcggagct ggcgagtcag ctggagtcca ctactgctgc gaagatgtca      600

gcggagcagg accgcgagaa cacgagggcc gcgctagagc agcagcttct cgaatccgag      660

gagcgcgctg gggagctggc gagtcagctg gagtccacta ctgctgcgaa gatgtcagcg      720

gagcaggacc gcgagaacac gagggccgcg ctagagcagc agcttcgtga ctccgaggag      780

cgcgctgcgg agctggcgag tcagctggag tccactactg ctgcgaagat gtcggcggag      840

caggaccgcg agaacacgag ggccacgcta gagcagcagc ttcgtgactc cgaggagcgc      900

gctgcggagc gtcga                                                      915

<210>  4
<211>  305
<212>  PRT
<213>  Leishmania donovani

<400>  4

His His His His His His Gly Ser Met Glu Leu Ala Thr Glu Trp Glu
1               5                   10                  15

Asp Ala Leu Arg Glu Arg Ala Leu Ala Glu Arg Asp Glu Ala Ala Ala
            20                  25                  30

Ala Glu Leu Asp Ala Ala Ala Ser Thr Ser Gln Asn Ala Arg Glu Ser
        35                  40                  45

Ala Ser Glu Arg Leu Thr Ser Leu Glu Gln Leu Leu Arg Glu Ser Glu
    50                  55                  60

Glu Arg Ala Gly Glu Leu Ala Ser Gln Leu Glu Ser Thr Thr Ala Ala
65                  70                  75                  80

Lys Met Ser Ala Glu Gln Asp Arg Glu Asn Thr Arg Ala Thr Leu Glu
                85                  90                  95

Gln Gln Leu Arg Asp Ser Glu Glu Arg Ala Ala Glu Leu Ala Ser Gln
            100                 105                 110

Leu Glu Ala Thr Ala Ala Ala Lys Ser Ser Ala Glu Gln Asp Arg Glu
        115                 120                 125

Asn Thr Arg Ala Ala Leu Glu Gln Gln Leu Arg Asp Ser Glu Glu Arg
    130                 135                 140

Ala Ala Glu Leu Ala Ser Gln Leu Glu Ser Thr Thr Ala Ala Lys Thr
145                 150                 155                 160

Ser Ala Glu Gln Asp Arg Glu Asn Thr Arg Ala Thr Leu Glu Gln Gln
                165                 170                 175

Leu Arg Asp Ser Glu Glu Arg Ala Ala Glu Leu Ala Ser Gln Leu Glu
            180                 185                 190

Ser Thr Thr Ala Ala Lys Met Ser Ala Glu Gln Asp Arg Glu Asn Thr
        195                 200                 205

Arg Ala Ala Leu Glu Gln Gln Leu Leu Glu Ser Glu Glu Arg Ala Gly
    210                 215                 220

Glu Leu Ala Ser Gln Leu Glu Ser Thr Thr Ala Ala Lys Met Ser Ala
225                 230                 235                 240

Glu Gln Asp Arg Glu Asn Thr Arg Ala Ala Leu Glu Gln Gln Leu Arg
                245                 250                 255

Asp Ser Glu Glu Arg Ala Ala Glu Leu Ala Ser Gln Leu Glu Ser Thr
            260                 265                 270

Thr Ala Ala Lys Met Ser Ala Glu Gln Asp Arg Glu Asn Thr Arg Ala
        275                 280                 285

Thr Leu Glu Gln Gln Leu Arg Asp Ser Glu Glu Arg Ala Ala Glu Arg
    290                 295                 300

Arg
305

<210>  5
<211>  22
<212>  DNA
<213>  Artificial Sequence

<220>
<223>  primer for cloning

<400>  5
gagctcgcaa ccgagtggga gg                                                22

<210>  6
<211>  17
<212>  DNA
<213>  Artificial Sequence

<220>
<223>  primer for cloning

<400>  6
gctccgcagc gcgctcc                                                      17


<210>  7
<211>  37
<212>  DNA
<213>  Artificial Sequence

<220>
<223>  primer for cloning

<400>  7
gtggaattct gcagatggat ccatggagct cgcaacc                                37


<210>  8
<211>  29
<212>  DNA
<213>  Artificial Sequence

<220>
<223>  primer for cloning

<400>  8
gccgccactg tgctggatgt cgacgctcc                                         29


<210>  9
<211>  117
<212>  DNA
<213>  Leishmania donovani

<400>  9
gagcagcagc ttcgtgactc cgaggagcgc gctgcggagc tggcgagtca gctggagtcc      60

actactgctg cgaagatgtc ggcggagcag gaccgcgaga acacgagggc cacgcta        117


<210>  10
<211>  117
<212>  DNA
<213>  Leishmania donovani

<400>  10
gagcagcagc ttcgtgactc cgaggagcgc gctgcggagc tggcgagcca gctggaggcc      60

actactgctg cgaagatgtc agcggagcag gaccgcgaga acacgagggc cacgcta        117

## Description

### Description and introduction to the general field of the invention

The invention refers to the diagnosis of leishmaniasis, in particular to the diagnosis of visceral leishmaniasis by the use of a new polypeptide from *Leishmania donovani* for an immunological rapid test.

According to a study of the "Special Programme for Research and Training in Tropical Diseases" (TRD) of the World Health Organization (WHO) from 2011, visceral leishmaniasis belongs to the most neglected diseases world-wide and particularly affects the poorest people in developing countries. About 500,000 new cases of illness arise each year, 90% of them occur only in six countries: India, Bangladesh, Nepal, the Sudan, Ethiopia and Brazil.

The disease is caused by protozoan parasites of the genus *Leishmania,* which cause a series of clinical pictures which are classified according to the mainly affected organs in visceral leishmaniasis (also referred to as kala-azar), cutaneous leishmaniasis and muco-cutaneous leishmaniasis. While cutaneous leishmaniasis is the most common form of leishmaniasis, visceral leishmaniasis is the most serious form in which the parasites migrate to vital organs, and is potentially fatal if untreated.

The pathogens are transmitted by sandfly (Phlebotominae), wherein animals, in particular dogs and small rodents, serve as intermediate host. 20 different human pathogenic *Leishmania* species are known, among them *L. chagasi, L. donovani, L. infantum, L. major, L. braziliensis* and *L. tropica.* Visceral leishmaniasis is caused by members of the L. *donovani* (*L. d*.) complex, comprising *L. d. donovani* in East Africa and the Indian subcontinent, *L. d. infantum* in Europe and North Africa and *L. d. chagasi* in Latin and South America (Lainson & Shaw 1987; Jamjoom et al. 2004). Recent studies show, however, that *L. chagasi* and *L. infantum* are very similar and can hardly be differentiated (Mauricio et al. 2000, Lukes et al. 2007; Alvar et al. 2012; PMID: 22693548).

Subclinical courses of visceral leishmaniasis and spontaneous remissions occur more frequently in *Leishmania infantum* and *Leishmania chagasi* infections than clinically manifest diseases. In contrast, *Leishmania donovani* infections are most frequently clinically manifest. If untreated, all diseases progressing as clinical visceral leishmaniasis comprise a high death rate. The high rate of *Leishmania* patients co-infected with HIV, malaria or other pathogens complicates the diagnosis and the course of the disease.

### State of the art

Conventionally, a most reliable diagnosis of visceral leishmaniasis is carried out via the direct detection of *Leishmania* parasite in organ aspirates. For this purpose, aspirates of spleen or liver tissue or of bone marrow need to be taken which requires trained medical personnel in hospitals which are rarely available in rural areas of developing countries. Since the 1990s, also non-invasive diagnostic procedures, such as the direct agglutination test (DAT) and immunochromatographic rapid tests are on the market. The direct agglutination test (Harith et al., Trans R Soc Trop Med Hyg. 1987; 81 (4):603-6) is very reliable, but can only be carried out in laboratories and requires approx. 10 hours until the result is available. Furthermore, the antigen has to be stored at 4-8°C. This is rather problematic in the endemic areas of Africa where temperatures may rise up to 45°C. The test is therefore not suitable for investigations by mobile physician teams who provide medical care in many regions of such countries.

The large advantage of the immunochromatographic rapid tests (dip-stick tests) consists of the fact that they can be carried out directly on-site without large efforts: Only a blood sample of the patient is required which can be examined by means of a test strip within short time. In these tests, usually a recombinant polypeptide fragment of the kinesin protein of *L. chagasi* (rk39) is used as a diagnostic tool which is immobilized on a test strip. This polypeptide contains an immunogenic motive of 39 repetitive amino acids and serves as an antigen for immunologic detection of *Leishmania* infection. Alternative tests use a homologous kinesin-related polypeptide of *Leishmania donovani* KE16 which is therefore referred to as rKE16. If an individual is infected by *Leishmania,* he most probably will develop antibodies against *Leishmania* polypeptides which can be detected in a blood or serum sample and will also bind to the antigen on the test strip.

The rk39 polypeptide is considered as being the best recombinant antigen for the immunological diagnosis; according to different literature sources, commercial tests achieve sensitivity and specificity values of 90-99% for detection of visceral leishmaniasis in Brazil and India. The tests available on the market, however, detect only 67-87% of the diseases in East Africa where leishmaniasis infections occur in Sudan before all, i.e. 13-33% of infected people in East Africa cannot be diagnosed with current rapid tests. As the disease predominantly occurs in rural areas lacking health care facilities, a reliable and simple diagnostic test is required particularly for this region, which can be performed without laboratory and clinical facilities. The WHO study "Visceral leishmaniasis Rapid Diagnostic Test Performance" from 2011 has explicitly pointed out the need for a better rapid test for East Africa (Cunningham et al., Clinical Infectious Diseases 2012(55); 10: 1312-9).

The patent US 6,375,955 B1 describes a polypeptide LmgSP9 from *L. major* which was cloned as an alternative antigen to rk39 for a more reliable diagnosis of leishmaniasis in patients in East Africa. Binding of this polypeptide to antibodies in the blood serum of confirmed leishmaniasis patients from Sudan and India was examined in an ELISA assay. Sera from healthy donors were used as negative controls. The ELISA was carried out with all sera and the known rk39 as antigen in parallel. The data shown in table 2 indicate that the polypeptide LmgSP9 reacts with some serum samples of *Leishmania* patients, which do not react with rk39. However, in other cases, infections which are clearly detected with rk39 are only weakly identified to be over the detection limit with LmgSP9. The values fluctuate considerably for the different patient sera, so that this polypeptide in fact is not suitable for the reliable detection of *Leishmania* infections, independent of region.

US 2007/0237794 A1 discloses another polypeptide, rLinJ16.1750r2, which was cloned from *L. infantum* for the reliable diagnosis of visceral leishmaniasis in Sudan. However, the data presented only show that rLinJ16.1750r2 is as reliable as the conventional rk39 when used for the immunologic detection of *Leishmania* infection in the serum of patients from Sudan. Thus, according to US 2007/0237794 A1 , 34 of 35 patient sera yield a positive result in an ELISA assay with rLinJ16.1750r2, while 35 of 35 of the sera were tested positive with rk39. Yet, especially those patients who cannot be diagnosed with rk39 require a more reliable diagnostic tool for visceral leishmaniasis in Sudan. Said patent application however does not disclose a polypeptide that allows a more reliable diagnosis of *Leishmania* infections in the countries of East Africa or the Sudan compared to the known rk39.

### Problem to be solved

One object of the present invention, therefore, is to provide an improved diagnostic tool for the detection of infections with pathogens of the genus *Leishmania,* in particular in individuals from Sudan or other East African countries.

### Solution to problem

According to the present invention, this object is achieved through a polypeptide, which contains an immunogenic part of the amino acid sequence comprising SEQ ID NO. 1 and causes a *Leishmania*-specific immune reaction. The invention refers to the diagnosis of leishmaniasis, in particular to the diagnosis of visceral leishmaniasis by the use of said new polypeptide from Leishmania donovani, in particular for an immunological rapid test. Accordingly, the present disclosure relates to a polypeptide sequence as set forth in SEQ ID NO: 1 and a polynucleotide sequence as set forth in SEQ ID NO. 2.

In another aspect of the invention, a vector is provided which carries a nucleotide sequence encoding a polypeptide according to the invention. According to further aspects of present invention DNA sequences encoding the polypeptides according to the invention are disclosed, recombinant expression vectors comprising these DNA sequences are provided and host cells transformed or transfected with such expression vectors are also disclosed.

Furthermore, the aim of the invention is achieved by providing a method for the detection of an infection with pathogens of the genus *Leishmania,* in which a biological sample from the individual to be examined is brought into contact with a polypeptide according to the present invention and the presence of antibodies to the polypeptide is detected in the biological sample, wherein the presence of antibodies to the polypeptide in the biological sample serves as evidence of a *Leishmania* infection.

In still another aspect a test kit is provided for the immunological detection of an infection with pathogens of the genus *Leishmania,* comprising a carrier, on which an inventive polypeptide is immobilised, and a detection reagent for the detection of antibodies from a biological sample to be examined bound to the polypeptide.

According to one aspect of present invention, a polypeptide is provided, wherein said peptide sequence is at least 98% identical to SEQ ID NO. 1. According to another aspect of present invention, a polypeptide is provided, wherein said peptide sequence is at least 99% identical to SEQ ID NO. 1, preferably is at least 100% identical to SEQ ID NO. 1. The polypeptide according to SEQ ID NO. 1 is subsequently also referred to as KLO8

The amino acid sequence of SEQ ID NO. 1 has 93% identity with the kinesin protein K39 from the *L. chagasi* strain BA-2 from Brazil (GenBank: AAA29254.1) and 88% identity with the kinesin-related protein KE16 from the *L. donovani* strain KE16 from India (GenBank: AAT40474.1), which are used in the known and commercially available diagnostic rapid tests to detect a *Leishmania* infection. Furthermore, the amino acid sequence of SEQ ID NO. 1 is 97% identical with the published Ldk39 protein of *L. donovani* strain 1 S-CL2D from Sudan (GenBank: ABI14928.1). The fact that there are still 3% sequence variations between these two proteins although derived from *Leishmania* strains from the same region, demonstrates that the amino acid sequence varies even between different strains from the same region.

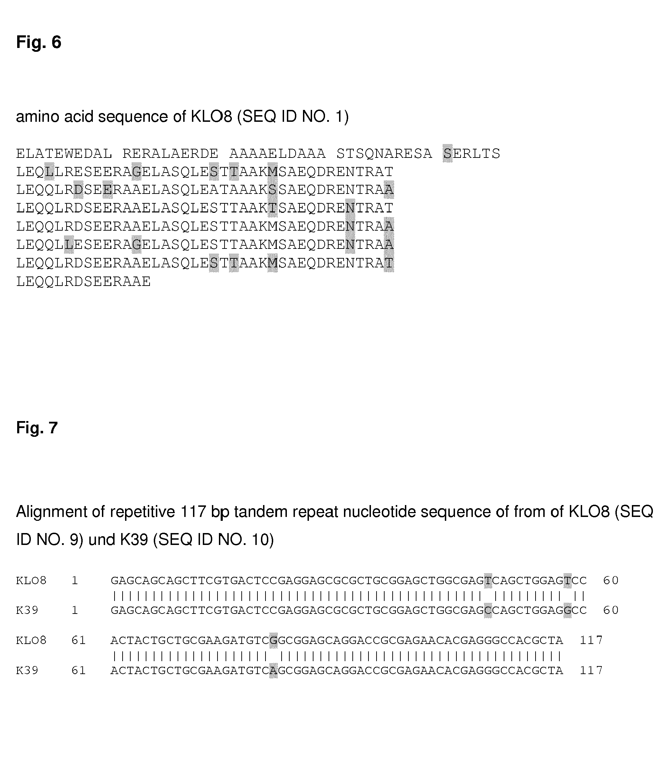

The amino acid sequence of polypeptide SEQ ID NO. 1 comprises an immunodominant tandem repeat sequence of repetitive 39 amino acids, which is present in the sequence according to SEQ ID NO. 1 in a 6.3-fold copy (see also Fig. 6). The marked positions within amino acid sequence of SEQ ID NO. 1 in Fig. 6 refer to variations compared to the consensus pattern of the immunodominant tandem repeat of published kinesin or kinesin-related proteins of *Leishmania* strains. One embodiment of present invention is directed to a polypeptide comprising at least one of said immunodominant tandem repeats. The at least one of said immunodominant tandem repeats is also indicated herein as "fragment".

Peptides according to current invention also include molecules which are distinguishable at one or more positions from the amino acid sequence of SEQ ID NO. 1 and have a high degree of homology to said sequence. Homology here means a sequence identity of at least 98 %, in particular an identity of 99 %, preferably over 99 %. The deviation to the amino acid sequences described above could arise through deletion, substitution and/or insertion.

The polypeptide KLO8 has according to one embodiment preferably a length of 294 amino acids according to a theoretical molecular mass of 32.4 kDa. Further experiments and recombinant expression of the polypeptide have demonstrated that it is particularly suitable to be used for the immunological detection of an infection with pathogens of the genus *Leishmania.* This is most particularly true for the diagnosis of infections in patients from East Africa or from Sudan. In this area, infected individuals cannot be detected with sufficient sensitivity by other known immunological tests. In contrast, *Leishmania* infections in Sudan can be very reliably diagnosed and differentiated diagnostically with high reliability from other diseases with similar symptoms using a polypeptide according to the present invention in a serological test.

One aspect of present invention relates to a nucleotide sequence comprising SEQ ID NO. 2. According to one embodiment, an isolated polynucleotide comprises a nucleotide sequence which is at least 90%, preferably at least 95% and most preferred at least 99% identical to SEQ ID NO. 2. The polypeptide sequence according to SEQ ID NO. 1 is preferably encoded by a nucleotide sequence comprising the 883 bp nucleotide sequence SEQ ID NO. 2. This sequence contains an immunodominant tandem repeat sequence of repetitive 117 bp, which is present in the sequence according to SEQ ID NO. 2 in a 6.3-fold copy. Said 117 bp sequences encode polypeptides with 39 amino acids each. An alignment of an immunodominant tandem repeat sequence of repetitive 117 bp from KLO8 und K39 is disclosed with Fig. 7.

The nucleotide sequence SEQ ID NO. 2 was amplified with PCR-primers designed according to the published sequences for the immunodominant areas of the kinesin protein k39 from *L. chagasi* on genomic DNA of promastigotes of the Leishmania donovani strain Lo8, which is an autochthonous Leishmania donovani strain in Sudan.

One aspect of present invention relates to a nucleotide sequence comprising SEQ ID NO. 2 without the nucleotide C at position 883. Said C is included within nucleotide sequence of SEQ ID NO. 2 as a result of amplification. Said nucleotide sequence comprising SEQ ID NO. 2 without the nucleotide C at position 883 represents the exact ORF for polypeptide according to SEQ ID NO. 1.

A polypeptide according to the present invention can be
- a polypeptide comprising the entire amino acid sequence of KLO8 as revealed in SEQ ID NO. 1,
- a polypeptide comprising a peptide sequence which is at least 98%, in particular 99% identical to SEQ ID NO. 1, or
- a polypeptide containing at least one fragment of said polypeptides.

In addition, polypeptides are within the scope of the invention only differing from the sequence of KLO8 by conservative substitutions or modifications.

Since the complete KLO8 recognises *Leishmania*-specific antibodies in biological samples from patients with very high sensitivity and specificity, it is preferred that the complete KLO8 polypeptide is used for diagnostic tests.

In principal, however, shorter fragments containing only parts of the KLO8 amino acid sequence according to SEQ ID NO. 1 can also be used. In particular, shorter fragments can be used which comprise a minor number of immunogenic repeats compared to SEQ ID NO. 1. Fragments of KLO8 are to be chosen in such a way that sensitivity and specificity of the test remain at a high level over a large group of infected and non-infected persons as well as individuals infected or co-infected with other diseases. This can be done by a person skilled in the art without leaving the range of protection of the claims.

Accordingly, another embodiment is directed to a polypeptide fragment derived from a peptide sequence as defined above. Yet another embodiment is directed to a polypeptide fragment which is at least about 39 amino acids in length derived from a peptide sequence as defined above. In still another embodiment a polypeptide fragment according to the present invention is encoded by at least one repeat sequence of 117 bp, which is present within the polynucleotide sequence according to SEQ ID NO. 2. The 117 bp repeats encode immunodominant peptide sequences. The 117 bp repeats are organized as tandem repeats within polynucleotide sequence according to SEQ ID NO. 2.

According to another embodiment polypeptides are provided comprising additional amino acids to the sequence of KLO8. Additional sequences can be inserted for example to facilitate the recombinant expression and subsequent purification of the polypeptide.

Very high values for sensitivity and specificity of the test can be achieved by one embodiment directed to a recombinant polypeptide according to SEQ ID NO. 3, which contains a his-tag and an additional Ser-Gly-Met which were inserted for cloning reasons at the N-terminus of the KLO8 sequence. Other additional peptide sequences can serve, for example, the immobilization of the polypeptide on a carrier substrate.

In one embodiment of the invention, the polypeptide comprises more than the 6.3 immunogenic repeats included in SEQ ID NO. 1.

If an individual is infected with *Leishmania,* the proteins of the pathogen evoke an immune response in the patient, wherein antibodies to strongly immunogenic pathogen peptides in particular are formed, including antibodies against the immunogenic areas of the kinesin protein. These in turn can be used to detect an infection, since the presence of antibodies to key pathogen polypeptides is indicative of an infection. Serologic testing is based on the presence of *Leishmania* specific antibodies. The reliability of such serological diagnostic method depends on how well the antibodies in biological samples of many different patients, who are infected with different types and strains of *Leishmania,* bind to the polypeptide used. *Leishmania* polypeptides used in the known immunological tests for the detection of *Leishmania* infection are reliably bound by antibodies in blood samples from patients from India and/or from South or Latin America. However, these tests have limited sensitivity for detection of antibodies formed by patients who are infected by *Leishmania donovani* strains from East Africa. A particular problem concerning serologic detection of *Leishmania* infection arises due to the fact that infected persons are often co-infected by HIV. These persons have a low titre of *Leishmania* specific antibodies which usually is below the detection limit of known tests so that these patients often are not recognised through known diagnostic methods. In contrast, polypeptides according to the present invention are very suitable antigens even for the reliable diagnosis of *Leishmania* infections in HIV patients.

One reason for the low sensitivity of known rapid tests in Sudan most probably is the finding that patients in Sudan have lower antibody titers against rk39 compared to individuals infected with *Leishmania* from India. A good diagnostic tool that also performs well in Sudan or other regions in East-Africa therefore seems to require an optimized polypeptide with very high affinity for antibodies produced in response to an infection with *Leishmania* in East-Africa. Polypeptides according to the present invention produce higher serologic test outputs compared to rk39. This is particularly true for ELISA assays.

Polypeptides within the scope of present invention comprise at least one immunogenic part of the amino acid sequence according to SEQ ID NO. 1. Polypeptides within the scope of present invention cause a *Leishmania*-specific immnune response.

In one embodiment of the invention, the polypeptide is used to detect visceral leishmaniasis. Visceral leishmaniasis is a particularly severe form leading to death of the patient if the disease is not diagnosed in an early stage and/or is not treated. Visceral leishmaniasis is caused by members of the *L. donovani* (*L. d*.) complex, comprising *L. d. donovani* in East Africa and the Indian subcontinent, *L. d. infantum* in Europe and North Africa and *L. d. chagasi* in Latin and South America. Therefore, according to one embodiment of the invention, the herewith presented polypeptides are used to detect an infection with *Leishmania donovani.*

In a diagnostic method for the detection of an infection with pathogens of the genus *Leishmania* according to the present invention the following steps are conducted:
- a biological sample of the individual to be examined is brought into contact with a polypeptide according to any one of the above disclosed embodiments and
- the presence of specific antibodies directed to a polypeptide according to any one of the above disclosed embodiments in said biological sample is detected, wherein the presence of said specific antibodies in the biological sample is indicative of a Leishmania infection.

With other words, a biological sample from the individual to be examined is brought into contact with a polypeptide according to the present invention and the presence of antibodies to the polypeptide in the biological sample is detected, wherein the presence of antibodies to the polypeptide in the biological sample serves as evidence of a *Leishmania* infection.

The term "sensitivity" of the test in this application is defined as the ratio of positive test results to the total number of serum samples from confirmed leishmaniasis patients tested. In other words, this is the proportion of actual positives which are correctly identified as such (e.g. the percentage of infected people who are correctly identified as being infected). "Specificity" refers to the ratio of negative test results to all tested serum samples from individuals, which have been proven to have no *Leishmania* infection. In other words, this is the proportion of negatives which are correctly identified (e.g. the percentage of healthy people who are correctly identified as not having the condition).

The biological sample can be any sample from the body of the individual to be examined that contains antibodies.

Preferably the biological sample is a blood sample, since blood samples are particularly simple to obtain and comprise high concentrations of antibodies. Serum samples in which the blood cells were removed by centrifugation e.g., are equally suitable, since the antibodies remain in the serum. Other suitable biological samples are lymph node aspirates, saliva, urine and plasma. However, the list of biological samples is not limited to these.

The individual to be examined can be a human. However, since animals serve as alternate hosts for *Leishmania* during transmission to humans and, like humans, also show an immune reaction to pathogen polypeptides, the method according to the invention can also be applied to animals for the detection of a *Leishmania* infection. Accordingly, one embodiment is directed to a method wherein the biological sample originates from a human or an animal. Among animals, *Leishmania* infections are especially relevant for dogs which live in close contact to humans and can be a source of infection also for humans. Furthermore, *Leishmania* infections also occur in other animals, in particular rodents.

The method for the detection of a *Leishmania* infection can be applied to humans or animals, for example dogs or rodents. The biological sample to be examined originates from a human or an animal. In the case of diagnosing *Leishmania* in animals, it has been shown that the method delivers very good results with dogs, in particular.

In principal, all immunological methods are suitable for the diagnosis of a *Leishmania* infection using a polypeptide according to the invention as antigen.

For example, *Leishmania* specific antibodies can be detected in biological samples of infected individuals using an ELISA assay in which the polypeptide is immobilised on a microtiter plate. Persons skilled in the art know how to coat a polypeptide onto ELISA microtiter plates as well as how to perform an ELISA assay. Likewise, immunochromatographic methods can be used, in which the polypeptide is immobilised on a limited area of a strip made from a special matrix material. The biological sample to be examined, preferably a liquid, is applied on one end of the strip and, due to capillary action, moves towards the other end. To enhance migration or reaction conditions, it can be of advantage to apply a running buffer in addition to the biological sample. Once the antibodies in the biological sample reach the location on which the polypeptide is bound, *Leishmania*-specific antibodies bind to the polypeptide and, thus, to the test strip, while the excess antibodies migrate out of the test strip. Bound antibodies are made visible by means of a suitable detection reagent, which can be present on the test strip or to be added separately. Suitable reagents and methods for making antigen-antibody-complexes visible are known by persons skilled in the art.

Suitable detection reagents are in particular anti-species secondary antibodies or protein A conjugates coupled with an indicator. Usually indicators are chosen which make the bound antibodies visible as coloured areas on the test strip. For example, colloidal gold particles, which show a positive test result as an orange-red colour line, are suitable as colour-giving indicators. Other indicators are, for example, fluorescent dyes or enzymatic activities which result in a colour or fluorescent reaction upon addition of a suitable substrate.

Another aspect of the present invention is directed to a carrier on which a polypeptide according to one of the disclosed embodiments is immobilised. According to one preferred embodiment of the method, the polypeptide is bound to a carrier. Most preferably, the polypeptide is immobilized onto a carrier which only has to be brought into contact with the biological sample to be examined and a suitable detection reagent. This is advantageous especially because such a diagnostic method is very easy to handle and can be easily carried out without the need for laboratory equipment or trained personnel.

In principal, all known materials, on which polypeptides can be immobilised, are suitable for being carriers, among them microtiter plates, nitrocellulose or other membranes onto which polypeptides adhere. Further carriers are glass or plastics, such as polyvinylchloride or polystyrene, which can also have a special coating for the binding of polypeptides. Methods through which polypeptides can be bound to such carriers are known by persons skilled in the art.

Polypeptides according to the invention can be bound to a carrier covalently or non-covalently by adsorption, for example. In order not to disrupt the structure of the polypeptide and, thus, the binding of *Leishmania* specific antibodies to the polypeptide, it is preferred to bind the polypeptide non-covalently to a carrier. Corresponding methods are known by persons skilled in the art.

In a preferred embodiment, the carrier is a test strip made of a matrix material which is suitable for immunochromatographic methods. Therefore, according to yet another embidiment a method is provided, wherein the carrier is a test strip made of a matrix material suitable for immunochromatographic methods. Such matrix materials are known by persons skilled in the art. Examples thereof are nitrocellulose, polysulfones and polycarboxylic acids. A detection method using an immunochromatographic test strip allows particularly simple application of the test at practically any location without requiring further materials, devices, or other laboratory equipment. Preferably, one such test strip comprises a suitable detection reagent besides the immobilised polypeptide for the detection of *Leishmania* specific antibodies bound to the immobilised polypeptide. Alternatively, the detection reagent can also be comprised in a separate reagent solution, which is applied together with the biological sample to the test strip. Furthermore, the detection reagent can be a developing solution, which is applied to the test strip after the passage of the sample.

In another embodiment of the invention, the carrier to which the polypeptide is bound is a porous membrane included in a flow-through device. Such membrane can be a nitrocellulose membrane or any other membrane to which polypeptides can be bound. To detect *Leishmania* specific antibodies in a biological sample, the sample is applied to the device and let flow through the membrane so that *Leishmania*-specific antibodies are trapped. After washing of the device and/or the membrane a detection reagent is applied to reveal the presence of bound antibodies indicative of an infection.

A method for the detection of an infection with pathogens of the genus *Leishmania* according to the present invention advantageously comprises the following steps:
■ Bringing the polypeptide bound to a carrier into contact with the biological sample,
■ Removing the excess, unbound part of the biological probe,
■ Bringing the polypeptide into contact with a reagent to detect binding of antibodies from the sample to the polypeptide,
■ Determining the test results through evaluation of the assay.

A reagent to detect binding of antibodies from the sample to the polypeptide can be applied onto the carrier in a separate step during the course of the test or it can already be present on the carrier. Protein A coupled with an indicator or anti-species second antibodies coupled with an indicator, which unspecifically bind to antibodies from the species from which the biological sample originated, are particularly preferred as detection reagents.

The determination of the test result can be qualitatively, through a colour reaction for example, or semi-quantitatively through comparison with standard values and/or cut off values. One embodiment is directed thus to a method, wherein the test result, i.e. the specific binding of antibodies from the biological sample to a polypeptide according to present invention, is determined by means of standard values and/or cut off values. When using a detection reagent producing a colour reaction, the appearance of colour indicates a positive result. Test methods, in which the detection reagent causes a colour reaction, are particularly practical, since they can be carried out very simply without special measuring devices and also without particular knowledge concerning test evaluation. If the detection reagent produces a colour reaction, it is preferable to provide a predefined colour scale to determine the test outcome, wherein only certain colours or colour intensities are indicative of a positive result. Furthermore, by evaluating the colour intensity or the absorption at a certain wavelength according to the detection reagent used, semi-quantitative test results can be obtained. In these cases, it is preferred to consider cut off values for example, which are identified by suitable preliminary investigations. How such cut off values can be sensibly identified and determined is known to persons skilled in the art. For example, negative cut off values can be obtained by using a population of healthy individuals and calculating a threshold limit from the statistical spread of test results achieved with biological samples of these individuals.

The object of the invention is furthermore achieved by a test kit for the immunological detection of an infection with pathogens of the genus *Leishmania.* According to one preferred embodiment, said test kit comprises a polypeptide according to the present invention.

According to another embodiment, said test kit comprises a carrier on which a polypeptide according to the present invention is immobilised.

According to yet another embodiment, said test kit further comprises a detection reagent for the detection of antibodies from a biological sample to be examined bound to the polypeptide.

Advantageously, a reaction buffer is additionally provided, which is particularly important in immunochromatographic tests to ensure sufficient liquid for the complete processing of the biological sample through the test strip. It is also preferred to include a reaction buffer in diagnostic test kits using other test formats, such as in the ELISA-format, in order to ensure optimal reaction conditions and minimal disruption of the test through impurities, introduced by solutions prepared by the user himself, for example.

Additionally, a comparison standard for cut off values and/or a positive control can also be included in the test kit. A positive control is particularly important, since it indicates the base functioning of the test, so that incorrect test results due to inadequate storage, incorrect usage, or malfunctioning detection reagent, for example, can be ruled out. For example, an anti-protein A antibody immobilised on the carrier can serve as a positive control in tests working with protein A coupled with an indicator as a detection reagent. Other possible positive controls are known by persons skilled in the art.

One embodiment of present invention is directed to a plasmid-vector comprising the nucleotide sequence SEQ ID NO. 2 or fragments thereof. A plasmid-vector can in one embodiment be used for recombinant expression of polypeptides according to the present invention, preferably a peptide comprising SEQ ID NO. 1 or fragments thereof. According to one embodiment, the plasmid-vector can be used for recombinant expression of polypeptides according to the present invention with a his-tag. Other types of tags are well known for the skilled person. According to a futher embodiment, the plasmid-vector comprises at least one of the 117 bp repeats of SEQ ID NO. 2. According to still another embodiment, the plasmid-vector comprises only one of the 117 bp repeats of SEQ ID NO. 2 for expression of one immunodominant peptide of polypeptide according to SEQ ID NO. 1.

Another embodiment of present invention is directed to a host cell comprising a recombinant vector as disclosed. A preferred embodiment of present invention is directed to host cells transformed or transfected with expression vectors comprising a nucleotide sequence SEQ ID NO. 2 or fragments thereof.

Another aspect of present invention is directed to the use of a polypeptide according to one of above disclosed embodiments for the immunological detection of an infection with pathogens of the genus Leishmania. Polypeptides according to the invention can be used for the serological diagnosis of infections with parasites of the genus *Leishmania.* In particular, the test is suitable to be used for the detection of infections with parasites of the *Leishmania donovani* complex. In another aspect, herewith disclosed polypeptides can also be used to stimulate an immune response against *Leishmania,* in particular against *Leishmania donovani,* in patients. In this aspect, the polypeptide according to the present invention can be used as a vaccine.

Percent (%) amino acid sequence identity or homology with respect to the polypeptide sequences identified herein is defined as the percentage of amino acid residues in a sequence that is identical with the amino acid residues in the polypeptide being compared, after aligning the sequences considering any conservative substitutions as part of the sequence identity. Alignment for purposes of determining percent amino acid sequence identity can be achieved in various ways that are within the skill in the art. Those skilled in the art can determine appropriate parameters for measuring alignment, including any algorithms needed to achieve maximal alignment over the full length of the sequences being compared.

### Embodiments

### Example 1 - Molecular cloning of the KLO8 gene fragment

The gene fragment encoding the immunodominant repeats of *L. donovani*strainLO8*,* designated *KLO8,* was amplified from genomic DNA from promastigotes. This is a reference strain of *L. donovani* from Sudan. The parasite was maintained in RPMI-1640 supplemented with L-glutamine, NaHCO₃ (Sigma-Aldrich) and 10% (v/v) fetal calf serum (Sigma-Aldrich) and genomic DNA was prepared according to standard protocols. The forward (5'-GAGCTCGCAACCGAGTGGGAGG-3') and reverse (5'- GCTCCGCAGCGCGCTCC-3') PCR primers (SEQ ID NO. 5 and 6 resp.) were designed according to the published *L. chagasi* gene for kinesin-related protein (GenBank: L07879.1). PCR reaction was performed using Phusion® High-Fidelity DNA Polymerase (FINNZYMES OY, Finland). Reaction was performed in a total of 50µl containing 3 % (v/v) DMSO, 10µl HF buffer, 10mM dNTPs mix-OLS (OMNI life science) and 100 ng genomic DNA. PCR was performed as follows: denaturation at 98°C for 30s followed by 25 cycles of denaturation at 98°C for 10s, annealing at 65.1 °C for 20 s, and extension at 72°C for 20 s. The amplified products revealed multiple bands with sizes equivalent to 117bp repeats. The largest amplification product (883bp) was gel purified, digested with *EcoR*V and subsequently cloned (according to the manufacturer's instructions) into the plasmid vector pcDNA3.1 (+) (Invitrogen life technologies, USA) resulting in the non-tagged KLO8 construct pcDNA/KLO8. The cloned sequence was confirmed by restriction digestion with *BamH*I and *Xba*I (Fermentas GmbH, Germany) and by sequence analysis. Each insert was sequenced at least twice. The sequence is identical to SEQ ID NO. 2. The deduced amino acid sequence corresponding to SEQ ID NO. 1 revealed that *KLO8* encodes a protein of 294 amino acids with a predicted molecular mass of 32.4 kDa and an isoelectric point (IP) of 4.39.

### Example 2 - Expression and purification of the recombinant polypeptide rKLO8 in E. coli

For expression and purification of the recombinant polypeptide, the DNA sequence encoding *KLO8* was subcloned into the his-tag vector pQE41 (Qiagen GmbH, Germany). The DNA construct pcDNA/KLO8 was used as template with the forward (5'-GTGGAATTCTGCAGATGGATCCATGGAGCTCGCAACC-3') and reverse (5'-GCCGCCACTGTGCTGGATGTCGACGCTCC-3') primers (SEQ ID NO. 7 and 8 resp.), designed to introduce *BamHI* and *SalI* restriction sites (underlined). Amplification was performed using Phusion® Hot Start II DNA Polymerase (Thermofisher Scientific, USA) as recommended by the manufacturer. Amplified DNA fragments were digested with the same restriction enzymes and cloned into the corresponding sites of the vector pQE41 to generate the plasmid construct pQE41/KLO8.

Sequence analysis of the cloned fragment revealed a partial open reading frame of a 883-basepair product. The DNA construct was further confirmed by restriction analysis using *BamHI* and *SalI* resulting in an approximately 883 bp product. SEQ ID NO. 3 discloses the entire DNA construct encoding the his-KLOB fusion protein.

To express the rKLO8-his-tag fusion protein disclosed in SEQ ID NO. 4, the plasmid pQE41/KLO8 was transformed into competent M15 *E. coli* cells (Qiagen GmbH, Germany). *E. coli* were grown at 37°C in Luria-Bertani (LB) medium containing 100 µg/ml ampicillin (Sigma-Aldrich, Germany) and 25 µg/ml kanamycin (Sigma-Aldrich, Germany) to the optical density (OD600) of 0.8. Recombinant protein expression was induced by adding 1mM isopropyl-β-D-thiogalactoside, IPTG (Roth, Germany) for 4 hours. *E. coli* cells were harvested by centrifugation at 3340xg for 10 min at 4°C. Bacterial pellets were then lysed in PBS (pH 7.4) containing 0,25 mg/ml lysozyme (Roth, Germany), 25 U/ml benzonase nuclease (Novagen, Germany), 10 mM imidazol (Roth, Germany), 1 mM PMSF (Sigma, USA) and 2µM β-mercaptoethanol (Sigma, USA). Subsequently, bacterial lysates were sonicated (Bandelin Sonorex, Germany) on ice 6 times for 10 seconds each with at least 10 seconds rest and stored at -20°C. The his-taggedrKLO8 fusion protein was recovered in the soluble fraction of the bacterial lysate as was demonstrated by SDS-PAGE. Purification was carried out using a nickel nitrilotriacetic (Ni-NTA) column (Qiagen GmbH, Germany). The supernatant was loaded into a Ni-NTA column pre-equilibrated with PBS (pH 7.4) containing 10 mM imidazole, 1 mM PMSF and 2µM β-mercaptoethanol. The recombinant protein was eluted with the same buffer containing 400 mM imidazole. Salts and imidazole were removed by dialysis in PBS buffer. Protein concentration was determined using Bradford assay against bovine serum albumin (BSA) as standard. Protein aliquots were kept frozen at -80°C.

### Example 3 - Bioinformatics analysis

The deduced amino acids sequence of the plasmid insert, determined with ExPASy Proteomics Server of Swiss Institute of Bioinformatics (http://web.expasy.org/translate/), was compared to the published sequences obtained from the National Centre for Biotechnology Information (http://www.ncbi.nlm.nih.gov/). Homology search was carried out using BLASTP 2.2.1. Two different clones were analyzed and found to contain the same insert (883bp). The identified DNA sequence was analyzed to locate and display tandem repeats using Tandem Repeats Finder (http://tandem.bu.edu/trf/trf.html) (Benson, 1999).

### Example 4 - SDS-Polyacrylamide gel electrophoresis (PAGE) and Western blot analysis

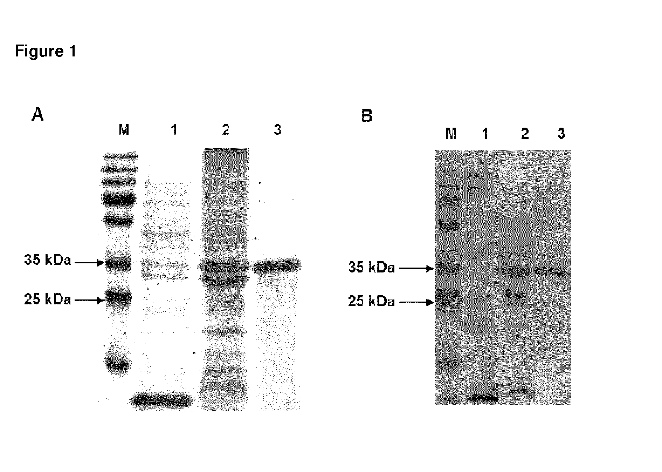

The recombinant protein rKLO8 was loaded on a 12% SDS-PAGE under denaturing conditions (Laemmli, 1970) using fractions of bacterial cell lysate or the purified protein and stained with Coomassie Brilliant Blue G250 (Merck KGaA, Germany). As shown in Fig. 1A (lane 2 & 3), the his-tagged fusion protein showed an apparent molecular weight of around 35kDa.

The reactivity of the recombinant protein KLO8 was assessed in Western blot analysis using pooled serum from 10 patients with confirmed *Leishmania donovani* infection from Sudan and pooled serum from 10 healthy controls from a non-endemic region in Sudan (see example 6 for details). Therefore, the resolved proteins were transferred to nitrocellulose transfer membrane (Whatman GmbH, Germany) using Bio-Rad Semi-dry Trans-Blot at 200 mA for 1 h. The membrane was then blocked with 5% BSA (w/v) in 100 mMNaCl, 0.05% Tween 20 (v/v) and 10 mMTris-HCl, pH 7.4 (blocking buffer) and then incubated for 18 h at 4°C with either patient or control sera diluted 1:1000 in blocking buffer. After washing, blots were incubated for 1 h at room temperature (RT) with Peroxidase-conjugated donkey anti-human IgG (H+L) (Jackson Immunoresearch Laboratories, USA) diluted 1:10000. The protein bands were revealed with Maximum Sensitivity Substrate system (Thermo Scientific, USA).As shown in Fig. 1 B, the positive serum recognized the recombinant KLO8 (lane 2&3) while the negative serum did not react with the polypeptide (lane 1). These results confirmed that the KLO8 is suitable for the specific detection of *Leishmania* antibodies present in individuals infected with the parasite.

### Example 5 - Establishing an ELISA for the detection of Leishmania specific antibodies in human serum samples

ELISAs were carried out using MaxiSorp™ high protein-binding capacity polystyrene 96 ELISA plates (NUNC TM Serving Life Science, Denmark). First, protein concentration for coating plates and optimal serum dilutions were determined in order to identify conditions which best discriminate between positive and negative sera. Therefore, pooled sera from 10 well characterized Sudanese patients with confirmed visceral leishmaniasis(with different antibody titres measured by DAT) and 10 pooled control sera from healthy individuals residing in non-endemic area in Sudan were used. Different protein concentrations were titrated against serial dilutions of positive or negative sera.

5 to 50 ng recombinant KLO8 per well were coated onto ELISA plates overnight at 4°C in 0,1 M NaCO3 buffer, pH 9,6. Plates were washed with PBS containing 0,05% (v/v) Tween-20 and then blocked with 3% (w/v) BSA in the same buffer at room temperature for 1 hour. After additional washes, 50 µl diluted positive or negative serum samples were added to each well, and plates were incubated at room temperature for 45 minutes. After washing, 50 µl/well Peroxidase-conjugated AffiniPure Donkey Anti-Human IgG (H+L) (Jackson Immunoresearch Laboratories, USA) diluted 1:10000 were added to each well and plates were incubated at room temperature for further 1 h. The reaction was visualised with hydrogen peroxide and tetramethyl benzidine (R&D Systems, USA). Reaction was stopped with 2N sulfuric acid after 10 minutes incubation in the dark. Optical density (OD) was measured at 450 nm using an ELISA microreader (FLUOstar Omega, BMG LABTECH). Each sample was tested in duplicates and at least twice and the mean value was calculated. Samples of invalid or inconsistent results were repeated.

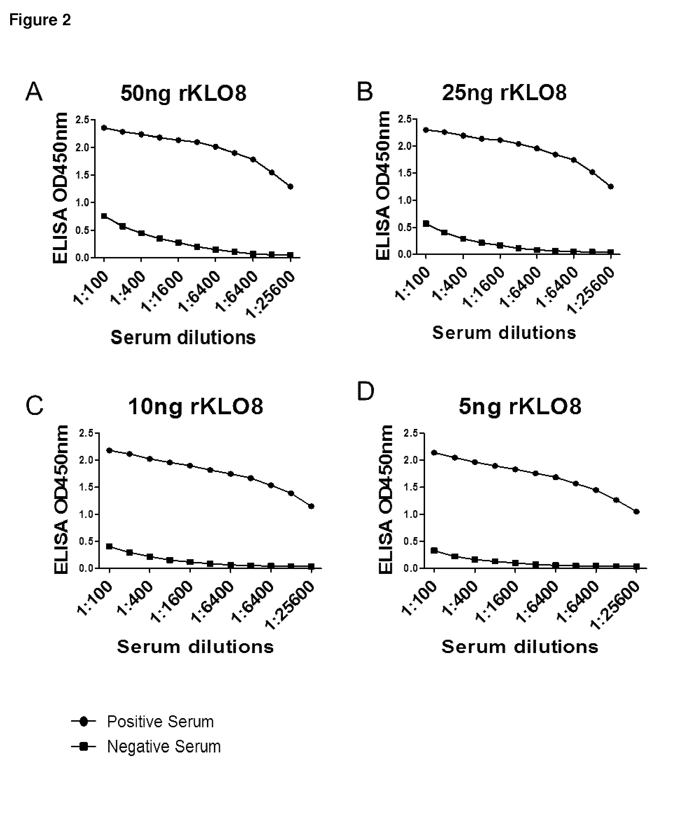

As shown in Fig. 2, all tested protein concentrations (5-50ng/well) were recognized by pooled serum of leishmaniasis patients and did not cross react with pooled serum from healthy individuals. ODs of positive serum were at least 4 fold higher compared to negative serum. Coating ELISA plates with a concentration of 5ngKLO8 protein was sufficient for positive detection of sera from patients infected with *Leishmania* diluted up to 1:25600. Thus, protein concentration of 5ng/well and serum dilutions of 1:800 was selected as conditions which provide optimal detection and were applied in subsequent experiments.

### Example 6 - Immunological detection of Leishmania donovani infection in blood serum of different individuals from Sudan via the KLO8 ELISA

A total number of 176 human serum samples serving as positive or negative probes were obtained from the serum bank at the Laboratory of Biomedical Research, Ahfad University (Omdurman, Sudan). 106 samples were from visceral leishmaniasis (VL) patients that were confirmed by lymph-node aspiration, 30 samples were from healthy individuals resident at Doka village (an endemic area for VL) and 10 from healthy people in the non-endemic area of Omdurman city. As further controls, sera from Sudanese patients suffering from other common diseases in the endemic areas were included in the study. 11 sera were from confirmed malaria cases, 10 from patients with diagnosed pulmonary tuberculosis and 6 from leukaemic patients. All sera were stored frozen (-20°C) at the Laboratory of Biomedical Research in Sudan. In addition, 10 control sera from uninfected German individuals were collected and included in the tests.

The ELISA was carried out as described in Example 5 with KLO8 polypeptides using sera from Sudanese individuals infected with *L. donovani* (n=106) and control (n=77) sera. Patients' and control sera were diluted 1:800 and tested on 5 ng KLO8 per well. As controls, the pooled positive and negative sera were included in each plate when testing individual sera.

Data were analyzed using GraphPad Prism software (GraphPad Prism Inc., San Diego, Ca). Significance of antibody responses was assessed using Student t test or one way ANOVA test. Differences of p-values < 0.05 were considered significant. The healthy controls from Sudan were used to determine the ELISA cut off value. These were defined for each recombinant protein as mean absorbance values of 40 sera of healthy controls from Sudan plus 3 standard deviations (SD).

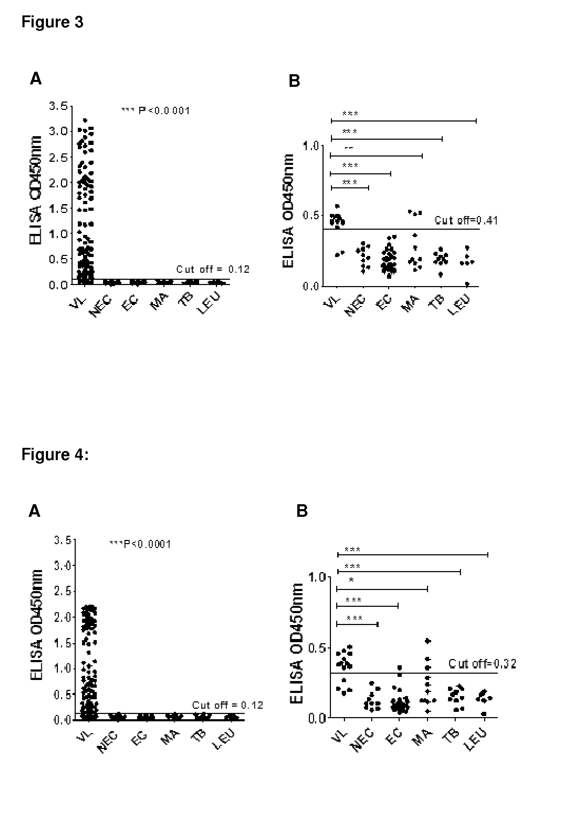

Table 1 shows all sera tested with their origins and characteristics. The results are depicted in figure 3 and table 2.

**Table 1: Sera from visceral leishmaniasis patients and controls from Sudan and Germany**

| **Origin (country)** | **No. ofsera** | **Clinical case** | | **Characteristics** | **Classification** |
|---|---|---|---|---|---|
| **Sudan** | 176 | VL | (n=106) | Diagnosed based on detection of parasite in stained LN smears. | confirmed VL patients |
| | | PKDL | (n=3) | Diagnosis was made clinically. | confirmed PKDL cases |
| | | NEC | (n=10) | From non-endemic area in Khartoum. | negative controls |
| | | EC | (n=30) | From the same VL- endemic area, Eastern Sudan. | |
| | | MA | (n=11) | Diagnosis was by demonstration of malaria parasite in blood smears. | |
| | | TB | (n=10) | Diagnosis was by detection of AFB of tuberculosis in sputum smears. | |
| | | LEU | (n=6) | Known cases of leukaemia. | |
| **Germany** | 10 | NEC | (n=10) | Non-endemic healthy volunteers from Germany. | |

| | | | | | |
|---|---|---|---|---|---|
| Abbreviations: VL, visceral leishmaniasis; PKDL, post kala-azar leishmaniasis; NEC, non-endemic controls; EC, endemic controls; MA, malaria; TB, tuberculosis; LEU, leukaemia; LN, lymph node; AFB, acid fast bacilli. | | | | | |

Quantitative analysis of antibodies in sera from visceral leishmaniasis patients demonstrated significantly higher antibody levels than those of control subjects (P<0.0001. Notably, none of the healthy or diseased controls (n=77) showed cross reaction with the recombinant KLO8 at serum dilutions of 1:800 (Fig. 3A). Indeed, malaria is known to be a major cause of cross reactivity to rK39 (Romero et al 2009, PMID: 19556562). Cross reactivity to rK39 has also been reported with healthy sera of endemic and non-endemic controls from Sudan (Pattabhi et al 2010, PMID: 20856856).

Sera from visceral leishmaniasis patients that had negative results (n=14) were re-tested at 1:100 serum dilution and compared to control sera (n=77). As shown in Fig. 3B, retesting on rKLO8 yielded increased positive detection of patients infected with *Leishmania* (12/14)

**Table 2:Immunological detection of Leishmania infection in blood serum of different individuals from Sudan via the rKLO8 ELISA compared to rk39 ELISA, the dipstick test and DAT**

| **Origin (country)** | **No. ofsera** | **Clinical case** | | **rKLo8 ELISA** | **rk39 ELISA** | **rk39 dip-sticktest** | **DAT** | **Test value** |
|---|---|---|---|---|---|---|---|---|
| | | | | No. positive results / total tested | | | | |
| **Sudan** | 176 | VL | n=106 | 104/106 | 102/106 | 86/106 | 100/106 | |
| | | | | 98.1% | 96.2% | 81.1% | 94.3% | sensitivity |
| | | PKDL | n=3 | 2/3 | 2/3 | 2/3 | 3/3 | |
| | | NEC* | n=10 | 0/10 | 0/10 | 0/10 | 0/10 | |
| | | EC* | n=30 | 0/30 | 1/30 | 0/30 | 0/30 | |
| | | MA* | n=11 | 3/11 | 3/11 | 1/11 | 0/11 | |
| | | TB* | n=10 | 0/10 | 0/10 | 0/10 | 0/10 | |
| | | LEU* | n=6 | 0/6 | 0/6 | 0/6 | 0/6 | |
| **Germany** | 10 | NEC* | n=10 | 0/10 | 0/10 | 0/10 | 0/10 | |
| | *: neg. ctrl. | | n=77 | 74/77 | 73/77 | 76/77 | 77/77 | |
| | | | | 96.1% | 94.8% | 98.7% | 100% | specificity |

| | | | | | | | | |
|---|---|---|---|---|---|---|---|---|
| Abbreviations: VL, visceral leishmaniasis; PKDL, post kala-azar leishmaniasis; NEC, non-endemic controls; EC, endemic controls; MA, malaria; TB, tuberculosis; LEU, leukaemia. | | | | | | | | |

### Example 7 - Diagnosis of visceral leishmaniasis in individuals from India and France

Sera were tested in an ELISA with rKLO8 polypeptide as described in example 6. Characteristics of sera tested are depicted in table 3. Results are shown in table 4.

The new KLO8 polypeptide was able to correctly detect 25 out of 26 (96,2%) *Leishmania* infections in sera from India and 26 out of 26 infections (100%) in sera from French patients. Notably, using KLO8 also 9 out of 11 *Leishmania* infections could be diagnosed in sera from patients co-infected with HIV.

**Table 3: Sera from visceral leishmaniasis patients and suspected cases from India and France**

| **Origin (country)** | **No. ofsera** | **Clinical case** | | **Characteristics** | **Classification** |
|---|---|---|---|---|---|
| **Bihar, North India** | 38 | VL | n=26 | Diagnosed based on detection of parasite in stained spleen smears. | confirmed VL patients |
| | | VLS | n=11 | Highly suspected symptomatic cases from Bihar, a known endemic area in North India, with negative spleen results. | |
| | | PKDL | n=1 | Diagnosis was made clinically | confirmed PKDL cases |
| **South France** | 75 | VL | n=26 | Diagnosed based on detection of parasite in stained BM smears and/or positive culture results. | confirmed VL patients |
| | | VL/HIV | n=11 | Patients with confirmed VL and HIV infection. | |
| | | VLS | n=13 | Highly suspected symptomatic cases from an endemic area in South France, with negative BM results | |
| | | ASC | n=25 | Asymptomatic cases with positive WB analysis results from a known VL-endemic area in South France. | |

| | | | | | |
|---|---|---|---|---|---|
| Abbreviations: VL, visceral leishmaniasis; VLS, VL suspects; PKDL, post kala-azar leishmaniasis; HIV, human immunodeficiency virus; VUHIV, VL/HIV co-infection; ASC, asymptomatic cases; BM, bone marrow, WB, Western blot. | | | | | |

In addition to the high sensitivity for detection of visceral leishmaniasis patients, the KLO8 ELISA has also the potential for detection of asymptomatic cases of leishmaniasis that are either subclinical cases and may develop the disease later or may remain subclinical but may act as a reservoir host for the disease transmission. Therefore, the test can also be used in epidemiological studies and help in designing control strategies for the disease.

**Table 4: Immunological detection of Leishmania infection in blood serum of different individuals from India and France via the rKLO8 ELISA compared to rk39 ELISA, the dipstick test and DAT. (All these sera (from India and France) were patients' sera, therefore, sensitivity can also be calculated for sera of VLS and ASC as well.)**

| **Origin (country)** | **No. of sera** | **Clinical case** | | **rKLo8 ELISA** | **rk39 ELISA** | **rk39 dipstick test** | **DAT** | **Test value** |
|---|---|---|---|---|---|---|---|---|
| | | | | No. positive results / total tested | | | | |
| **Bihar, North India** | 38 | VL | (n=26) | 25/26 | 25/26 | 25/26 | 24/26 | |
| | | | | 96.2% | 96.2% | 96.2% | 92.3% | sensitivity |
| | | VLS | (n=11) | 9/11 | 9/11 | 9/11 | 9/11 | |
| | | PKDL | (n=1) | 1/1 | 1/1 | 1/1 | 1/1 | |
| **South France** | 75 | VL | (n=26) | 26/26 | 26/26 | 23/26 | 23/26 | |
| | | | | 100% | 100% | 88.5% | 88.5% | sensitivity |
| | | VL/HIV | (n=11) | 9/11 | 9/11 | 9/11 | 6/11 | |
| | | | | 81.8% | 81.8% | 81.8% | 54.5% | sensitivity |
| | | VLS | (n=13) | 10/13 | 10/13 | 10/13 | 10/13 | |
| | | ASC | (n=25) | 7/25 | 3/25 | 3/25 | 1/25 | |

| | | | | | | | | |
|---|---|---|---|---|---|---|---|---|
| Abbreviations: VL, visceral leishmaniasis; VLS, VL suspects; PKDL, post kala-azar leishmaniasis; HIV, human immunodeficiency virus; VUHIV, VL/HIV co-infection; ASC, asymptomatic cases; BM, bone marrow. | | | | | | | | |

### Comparative Example 1 - Immunological detection of Leishmania infection in blood sera of different individuals using the known rk39 polypeptide in a comparative ELISA

Recombinant rk39 polypeptide of *Leishmania chagasi* according to GenBank accession number AAA29254.1 was purchased from Rekom Biotech, S.L., Granada, Spain. It was expressed as a fusion protein with a 6 x his-tag at the C-terminus in *E. coli.* Upon received, the protein concentration was verified with the same method used to measure the recombinant protein rKLO8 (Bradford). Aliquots were kept at -80°C.

As described in the inventive examples 6 and 7, ELISAs were carried out with the same serum samples in plates coated with 5 ng/well rk39.

The results are depicted in figure 4 and tables 2 and 4.

### Comparative Example 2 - rk39 dipstick test

Individual IT LEISH dipstick kits for antibody detection in human visceral leishmaniasis were purchased from Bio-Rad, France, which use the recombinant rk39 antigen (Burns et al., 1993). The test was performed and interpreted as recommended by the manufacturer. Sera were considered positive when a dark purple control band appeared. Samples with invalid results were repeated. Results are shown in tables 2 and 4.

### Comparative Example 3 - Direct agglutination test (DAT)

The DAT (ITMA-DAT/VL) kits (Lot 11D1B1) were purchased from Institute of Tropical Medicine, Antwerp-Belgium (ITMA). The antigen is a freeze-dried suspension of trypsin-treated, fixed and stained promastigotes of *L. donovani* strain 1-S (Harith et al., 1988; Meredith et al., 1995). The test was performed in 96 V-shaped microplates (Greiner Bio One, Germany) according to the manufacturer's instructions. In addition to the control wells, positive and negative pooled sera were included in each plate and the results were read after overnight incubation at room temperature. Samples with titres equal to or higher than 1:3200 were considered positive whereas samples with titres between 1:800 and 1:1600 were considered as borderline and were repeated. Results are shown in tables 2 and 4.

### Example 8 - Comparison of diagnostic value of the rKLO8 ELISA and the dipstick test or direct agglutination test

For data obtained with sera from Sudan, sensitivity, specificity, positive predictive value (PPV) and negative predictive value (NPV) were calculated to assess the usefulness of the diagnostic assays. Sensitivity was defined as the percentage of the serum samples of confirmed patients infected with *Leishmania* (n=106) with positive test results. Specificity was assessed as percentage of total serum samples from non-VL cases (n=77) with negative test results. This group included healthy endemic controls, malaria, TB, and leu-kemic patients. PPV was calculated as number of true positives divided by the number of true positives and false positives. NPV was calculated as number of true negatives divided by the number of true negatives plus number of false negatives.

Calculated values for sensitivity, specificity, negative and positive predictive values of the three tests in Sudan are depicted in table 5.

**Table 5: Diagnostic performance of rKLO8 ELISA, rK39 ELISA, rK39 dipstick test and DAT for detection of visceral leishmaniasis in Sudan**

| | rKLO8 ELISA | rK39 | | DAT |
|---|---|---|---|---|
| | | ELISA | dipstick test | |
| Sensitivity* | 98.1% | 96.2% | 81.1% | 94.3% |
| Sensitivity (1:800)** | 92.5% | 86.8% | | |
| Specificity* | 96.1% | 94.8% | 98.7% | 100% |
| PPV | 97.2% | 97.2% | 98.9% | 100% |
| NPV | 97.4% | 94.8% | 79.2% | 92.8% |

| | | | | |
|---|---|---|---|---|
| Abbreviations: PPV, positive predictive value; NPV, negative predictive value. * The overall sensitivity and specificity of rKLO8- or rK39 ELISA were calculated as the combined results obtained at serum dilutions, 1:800 or 1:100. ** Sensitivity of ELISAs using KLO8 an rk39 at serum dilutions of 1:800. | | | | |

Results obtained from rKLO8 ELISA were compared with two commercial diagnostic kits, the rk39 strip test (Bio-Rad) and a freeze-dried version of DAT (ITMA-DAT/VL), as well as an rk39 ELISA using the same panel of VL and control sera. In general, sera tested in KLO8 ELISA had higher OD405 values than in the rk39 ELISA with a mean value of 1.12+0.96 for rKLO8 and 0.92+0.76 for rk39.This is of practical importance since increased sensitivity helps to discriminate between potentially cross reactive sera.

The data obtained clearly indicate that the new recombinant KLO8 polypeptide can be used for the diagnosis of visceral leishmaniasis in patients from Sudan with clearly higher sensitivity than the known tests. As shown in table 5, the overall sensitivity of rKLO8 (98.1%) was higher than that of rk39 (96.2%) when measured by ELISA, while the rk39 dipstick test yielded only 81.1% and DAT 94.3%. With respect to specificity, the rKLO8 ELISA showed better performance (96.1%) compared to rk39 ELISA (94.8%) but lower than DAT (100%) and the rk39 dipstick test (98.7%). This is due to the fact that some sera from malaria patients were tested positive in the rKLO8 ELISA although these patients were not infected with *Leishmania.* Accordingly, the PPVs and NPVs were 97.2% and 97.4% for rKLO8 ELISA, 97.2% and 94.8% for rK39 ELISA, 98.9% and 79.2% for rK39 strip test and 100% and 92.8% for DAT, respectively. However, test sensitivity is the most important factor for a rapid diagnostic test to be used in the field, i.e. the most reliable recognition of infected individuals since non-detected *Leishmania* infection might lead to death of the patient. Although false positive test results should be omitted, for the purpose of detection of *Leishmania* infection in the rural areas of East Africa it is better to have a higher sensitivity (high rate of true positive results) than a maximum specificity (minimum number of false positives).

Therefore, the KLO8 polypeptide is superior to commonly used serological tests with regard to immunological diagnosis of *Leishmania* infection. While having a comparably high sensitivity and specificity for India and France, it works better than the known tests for individuals from Sudan and most probably also from other regions of East-Africa where similar strains of *Leishmania donovani* exist. The KLO8 polypeptide is a most promising candidate antigen for serving as a diagnostic tool to detect *Leishmania* infections all over the world with very high sensitivity and specificity.

### Example 9 - Comparing the reactivity of recombinant polypeptides KLO8 and rk39 with visceral leishmaniasis sera from various endemic regions

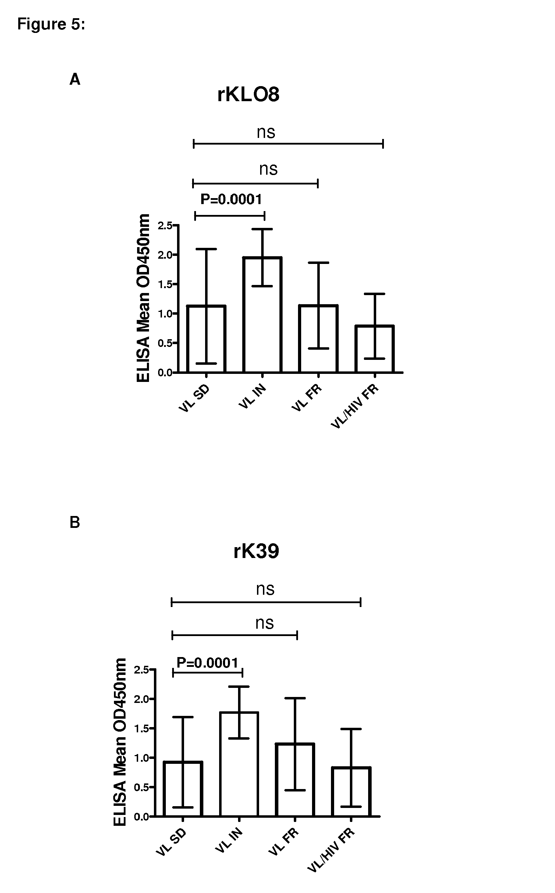

ELISAs using KLO8 or the rk39 polypeptide were performed as described in example 5, 6, 7 and comparative example 1. Sera from visceral leishmaniasis patients from Sudan, India and France as well as sera from French HIV and Leishmania co-infected patients were analysed. The mean OD 450 values were calculated and compared by statistical analysis. The results are shown in table 6 and figure 5.

**Table 6: Mean ELISA OD 450 values using KLO8 and rk39 as antigen**

| Origin of sera- clinical case | rKLO8 ELISA mean±SD | rK39 ELISA mean±SD |
|---|---|---|
| Sudan-VL (n=1 06) | 1.12±0.96 | 0.92±0.76 |
| India-VL (n=26) | 1.94±0.48 | 1.76±0.44 |
| France-VL (n=26) | 1.13±0.72 | 1.23±0.78 |
| France-VL/HIV (n=11) | 0.78±0.54 | 0.83±0.66 |

| | | |
|---|---|---|
| Abbreviations: VL, visceral leishmaniasis; HIV, human immunodeficiency virus; VUHIV, VL/HIV co-infection; SD, standard deviation. | | |

While sera from infected individuals from India show a high reactivity and a high OD values in both ELISA tests, sera from leishmaniasis patients from Sudan only have a mean OD value that is reduced by approximately 50%. This tendency is true for both KLO8 and rk39 ELISA.

However, concerning patients from Sudan the reactivity of KLO8 is clearly higher than that of rk39 (mean+SD; 1.12+0.97 versus 0.92+0.77). Moreover, the KLO8 was significantly more sensitive for the detection of visceral leishmaniasis in patients from Sudan than rk39 when challenged at high serum dilutions (1:800); the sensitivity values calculated were 86.8% for rk39 compared to 92.5% for KLO8. As a result of the increased reactivity KLO8 can be expected to be more sensitive than rk39 in a rapid test format like a dip stick test, for example.

### Example 10-Detection of Leishmania infection in sera of dogs from known endemic regions in Portugal and Brazil

To determine the efficiency of the recombinant polypeptide KLO8 for the detection of *Leishmania* antibodies in dogs having canine form of visceral leishmaniasis, 43 sera from known endemic regions for canine visceral leishmaniasis in Portugal and Brazil were analysed. In addition, 20 sera from non-infected asymptomatic healthy dogs from Croatia were used to determine the cut-off value, which was found to be 0.12. The ELISA was optimised using the same protein concentration (5ng/well) and serum dilutions (1:800) as described in Example 5 for human sera. The ELISA protocol followed for the analysis of dogs' sera was as described in example 5 except for using rabbit anti-dog IgG (H+L) as a secondary antibody (JaksonlmmunoResearch Laboratories, INC.). An overview of the sera tested is given in table 7. The ELISA results were compared to outcome of the direct agglutination test (DAT). Results are depicted in table 8.

As shown for humans, also dogs infected with *Leishmania* can be diagnosed with very high sensitivity via immunologic tests using the KLO8 polypeptide as an antigen. 100% of confirmed cases of canine visceral leishmaniasis from Portugal were recognized correctly by both KLO8 ELISA and DAT. In addition, also a high proportion of highly suspected symptomatic or asymptomatic cases yielded positive test results when analysed by KLO8 ELISA. Both symptomatic and asymptomatic cases were obtained from known canine leishmaniasis foci in Portugal and Brazil.

**Table 7: Sera of dogs with canine visceral leishmaniasis and control sera**

| **Origin (country)** | **No. of sera** | **Clinical case** | | **Characteristics** | **Infecting parasite** |
|---|---|---|---|---|---|
| **Évora, South Portugal** | 27 | CVL | n=9 | Confirmed CVL diagnosed based on detection of the parasite in stained organ smears. | *L. infantum* |
| | | ASC | n=18 | Asymptomatic dogs from the same endemic region in south Portugal. | |
| **Caicó Paraíba, Brasil** | 16 | SC | n=3 | Symptomatic dogs with positive ELISA using the recombinant HSP. | *L. chagasi* |
| | | ASC | n=13 | Asymptomatic dogs with positive ELISA using HSP. | |
| **Croatia** | 20 | ASC | n=20 | Asymptomatic healthy dogs with negative IFAT results. | |

| | | | | | |
|---|---|---|---|---|---|
| Abbreviations: CVL, canine visceral leishmaniais; ASC, asymptomatic cases; SC, symptomatic cases; HSP, heat shock protein; IFAT, immunofluorescence antibody test. | | | | | |

**Table 8: Immunological detection of Leishmania infection in blood serum of infected dogs from Portugal and Brazil and control sera from Croatia via the rKLO8 ELISA compared to DAT**

| **Origin (country)** | **No. of sera** | **Clinical case** | | **rKLO8 ELISA** | **DAT** |
|---|---|---|---|---|---|
| | | | | No. tested positive/total | |
| **Évora, South Portugal** | 27 | CVL | (n=9) | 9/9 | 9/9 |
| | | ASC | (n=18) | 7/18 | 6/18 |
| **Caicó Paraíba, Brasil** | 16 | SC | (n=3) | 2/3 | 2/3 |
| | | ASC | (n=13) | 9/13 | 4/13 |
| **Croatia** | 20 | ASC | (n=20) | 0/20 | 0/20 |

| | | | | | |
|---|---|---|---|---|---|
| Abbreviations: CVL, canine visceral leishmaniais; ASC, asymptomatic cases; SC, symptomatic cases. | | | | | |

As observed for human sera from asymptomatic cases described in example 7, the KLO8 ELISA also has the potential for detection of infected asymptomatic dogs, which consider to be the main reservoir host for leishmaniasis. In fact, only a part of infected clinically healthy dogs can develop clinical disease while the rest will remain asymptomatic and will never develop clinical signs. However, this group will act as the main reservoir of infection and therefore should be detected and treated in order to limit the transmission of the infection.

### Figure Legends and Abbreviations

- Fig. 1A:: The recombinant protein rKLO8 was loaded on a 12% SDS-PAGE under denaturing conditions using fractions of bacterial cell lysate or the purified protein and stained with Coomassie Brilliant Blue G250 (Merck KGaA, Germany). As shown in lanes 2 & 3, the his-tagged fusion protein shows an apparent molecular weight of around 35 kDa.
- Fig. 1B:: The reactivity of the recombinant protein KLO8 was assessed in Western blot analysis using pooled serum from 10 patients with confirmed Leishmania donovani infection from Sudan and pooled serum from 10 healthy controls from a non-endemic region in Sudan. The resolved proteins were transferred to nitrocellulose transfer membrane, the membrane was blocked and then incubated with either patient or control sera. After washing, blots were incubated with Peroxidase-conjugated donkey anti-human IgG (H+L). The protein bands were revealed with Maximum Sensitivity Substrate system. The positive serum recognized the recombinant KLO8 (lane 2 & 3) while the negative serum did not react with the polypeptide (lane 1).
- Fig. 2:: All tested protein concentrations (5-50 ng/well) were recognized by pooled serum of leishmaniasis patients and did not cross react with pooled serum from healthy individuals (A - D). ODs of positive serum were at least 4 fold higher compared to negative serum. Coating ELISA plates with a concentration of 5 ng KLO8 protein was sufficient for positive detection of sera from patients infected with Leishmania diluted up to 1:25600.
- Fig. 3:: KLO8 ELISA results with patients and control sera diluted 1:800 (A) and 1:100 (B). Reactivity were challenged using panels of individuals VL patients' (n=106) or healthy (n=77) sera including non-endemic healthy controls, NEC (20), endemic healthy controls, EC (30), malaria, MA (n=11), tuberculosis, TB (n=10), or leukemia, LEU (n=6).
- Fig. 4:: rk39 ELISA results with patients and control sera diluted 1:800 (A) and 1:100 (B). Reactivity were challenged using panels of individuals VL patients' (n=106) or healthy (n=77) sera including non-endemic healthy controls, NEC (20), endemic healthy controls, EC (30), malaria, MA (n=11), tuberculosis, TB (n=10), or leukemia, LEU (n=6).
- Fig. 5:: Mean OD 450 values as determined by the KLO8 (A) and rk39 ELISA (B) respectively tested at 1:800 serum dilutions. Mean values were calculated from all results obtained with sera described in examples 6 and 7 (KLO8 ELISA) and in comparative example 1 (rk39 ELISA). Significance was calculated at p=0,0001 level. Abbreviations: VL, visceral leishmaniasis; SD, Sudan; IN, India; FR, France; HIV, human immunodeficiency virus; VL/HIV, VL/HIV co-infection; ns, not significant, p, statistical p-value.
- Fig. 6:: The amino acid sequence of SEQ ID NO. 1 comprises an immunodominant tandem repeat sequence of repetitive 39 amino acids, which is present in the sequence according to SEQ ID NO. 1.
- Fig. 7:: Alignment of an immunodominant tandem repeat sequence of repetitive 117 bp from KLO8 (SEQ ID NO. 9) and K39 (SEQ ID NO. 10).

Sequences

SEQ ID NO. 1: (amino acid sequence)
ELATEWEDAL RERALAERDE AAAAELDAAA STSQNARESA SERLTSLEQL
LRESEERAGE LASQLESTTA AKMSAEQDRE NTRATLEQQL RDSEERAAEL
ASQLEATAAA KSSAEQDREN TRAALEQQLR DSEERAAELA SQLESTTAAK
TSAEQDRENT RATLEQQLRD SEERAAELAS QLESTTAAKM SAEQDRENTR
AALEQQLLES EERAGELASQ LESTTAAKMS AEQDRENTRA ALEQQLRDSE
ERAAELASQL ESTTAAKMSA EQDRENTRAT LEQQLRDSEE RAAE

SEQ ID NO. 2: (DNA sequence)
GAGCTCGCAA CCGAGTGGGA GGACGCACTC CGCGAGCGTG
CGCTTGCAGA GCGTGACGAA GCCGCTGCAG CCGAACTTGA
TGCCGCAGCC TCTACTTCCC AAAACGCACG TGAAAGCGCC
TCCGAGCGGC TAACCAGCCT TGAGCAGCTG CTTCGCGAAT
CCGAGGAGCG CGCTGGGGAG CTGGCGAGTC AGCTGGAGTC
CACTACTGCT GCGAAGATGT CGGCGGAGCA GGACCGCGAG
AACACGAGGG CCACGCTAGA GCAGCAGCTT CGTGACTCCG
AGGAGCGCGC TGCGGAGCTG GCGAGCCAGC TGGAGGCCAC
TGCTGCTGCG AAGTCGTCGG CGGAGCAGGA CCGCGAGAAC
ACGAGGGCCG CGTTGGAGCA GCAGCTTCGT GACTCCGAGG
AGCGCGCTGC GGAGCTGGCG AGTCAGCTGG AGTCCACTAC
TGCTGCGAAG ACGTCGGCGG AGCAGGACCG CGAGAACACG
AGGGCCACGC TAGAGCAGCA GCTTCGTGAC TCCGAGGAGC
GCGCTGCGGA GCTGGCGAGT CAGCTGGAGT CCACTACTGC
TGCGAAGATG TCAGCGGAGC AGGACCGCGA GAACACGAGG
GCCGCGCTAG AGCAGCAGCT TCTCGAATCC GAGGAGCGCG
CTGGGGAGCT GGCGAGTCAG CTGGAGTCCA CTACTGCTGC
GAAGATGTCA GCGGAGCAGG ACCGCGAGAA CACGAGGGCC
GCGCTAGAGC AGCAGCTTCG TGACTCCGAG GAGCGCGCTG
CGGAGCTGGC GAGTCAGCTG GAGTCCACTA CTGCTGCGAA
GATGTCGGCG GAGCAGGACC GCGAGAACAC GAGGGCCACG

CTAGAGCAGC AGCTTCGTGA CTCCGAGGAG CGCGCTGCGG
AGC

SEQ ID NO. 3: (nucleotide sequence of recombinant his-KLO8)
CATCACCATCACCATCACGGATCCATGGAGCTCGCAACCGAGTGGGAGGACGCACTCCGCG
AGCGTGCGCTTGCAGAGCGTGACGAAGCCGCTGCAGCCGAACTTGATGCCGCAGCCTCTAC
TTCCCAAAACGCACGTGAAAGCGCCTCCGAGCGGCTAACCAGCCTTGAGCAGCTGCTTCGC
GAATCCGAGGAGCGCGCTGGGGAGCTGGCGAGTCAGCTGGAGTCCACTACTGCTGCGAAGA
TGTCGGCGGAGCAGGACCGCGAGAACACGAGGGCCACGCTAGAGCAGCAGCTTCGTGACTC
CGAGGAGCGCGCTGCGGAGCTGGCGAGCCAGCTGGAGGCCACTGCTGCTGCGAAGTCGTCG
GCGGAGCAGGACCGCGAGAACACGAGGGCCGCGTTGGAGCAGCAGCTTCGTGACTCCGAGG
AGCGCGCTGCGGAGCTGGCGAGTCAGCTGGAGTCCACTACTGCTGCGAAGACGTCGGCGGA
GCAGGACCGCGAGAACACGAGGGCCACGCTAGAGCAGCAGCTTCGTGACTCCGAGGAGCGC
GCTGCGGAGCTGGCGAGTCAGCTGGAGTCCACTACTGCTGCGAAGATGTCAGCGGAGCAGG
ACCGCGAGAACACGAGGGCCGCGCTAGAGCAGCAGCTTCTCGAATCCGAGGAGCGCGCTGG
GGAGCTGGCGAGTCAGCTGGAGTCCACTACTGCTGCGAAGATGTCAGCGGAGCAGGACCGC
GAGAACACGAGGGCCGCGCTAGAGCAGCAGCTTCGTGACTCCGAGGAGCGCGCTGCGGAGC
TGGCGAGTCAGCTGGAGTCCACTACTGCTGCGAAGATGTCGGCGGAGCAGGACCGCGAGAA
CACGAGGGCCACGCTAGAGCAGCAGCTTCGTGACTCCGAGGAGCGCGCTGCGGAGCGTCGA
C

SEQ ID NO. 4: (amino acid sequence of recombinant 6xhis-KLO8)
HHHHHHGSMELATEWEDALRERALAERDEAAAAELDAAASTSQNARESASERLTSLEQLL
RESEERAGELASQLESTTAAKMSAEQDRENTRATLEQQLRDSEERAAELASQLEATAAAK
SSAEQDRENTRAALEQQLRDSEERAAELASQLESTTAAKTSAEQDRENTRATLEQQLRDS
EERAAELASQLESTTAAKMSAEQDRENTRAALEQQLLESEERAGELASQLESTTAAKMSA
EQDRENTRAALEQQLRDSEERAAELASQLESTTAAKMSAEQDRENTRATLEQQLRDSEERA
AERR

SEQ ID NO. 5: (Nucleotide sequence of 5' PCR primer for cloning of KLO8)
GAGCTCGCAACCGAGTGGGAGG

SEQ ID NO. 6: (Nucleotide sequence of 3' PCR primer for cloning of KLO8)
GCTCCGCAGCGCTCC

SEQ ID NO. 7: (Nucleotide sequence of 5' PCT primer for subcloning of KLO8 in pQE41)
GTGGAATTCTGCAGATGGATCCATGGAGCTCGCAACC

SEQ ID NO. 8: (Nucleotide sequence of 3' PCT primer for subcloning of KLO8 in pQE41)
GCCGCCACTGTGCTGGATGTCGACGCTCC

SEQ ID NO. 9: Repetitive 117 bp tandem repeat nucleotide sequence of from of KLO8
GAGCAGCAGCTTCGTGACTCCGAGGAGCGCGCTGCGGAGCTGGCGAGTCAGCTGGAGTCC
ACTACTGCTGCGAAGATGTCGGCGGAGCAGGACCGCGAGAACACGAGGGCCACGCTA

SEQ ID NO. 10: Repetitive 117 bp tandem repeat nucleotide sequence of from of K39
GAGCAGCAGCTTCGTGACTCCGAGGAGCGCGCTGCGGAGCTGGCGAGCCAGCTGGAGGCC
ACTACTGCTGCGAAGATGTCAGCGGAGCAGGACCGCGAGAACACGAGGGCCACGCTA

## Claims

1. A polypeptide comprising a peptide sequence at least 98% identical to SEQ ID NO. 1.

2. The polypeptide according to claim 1 wherein said peptide sequence is at least 99% identical to SEQ ID NO. 1, preferably 100% identical to SEQ ID NO. 1.

3. A polypeptide fragment which is at least about 39 amino acids in length derived from a peptide sequence as defined in claim 1 or claim 2.

4. An isolated polynucleotide comprising a nucleotide sequence of at least 90% identity to SEQ ID NO. 2.

5. The isolated polynucleotide according to claim 4 comprising a nucleotide sequence of at least 95%, in particular 99%, preferably 100% identity to SEQ ID NO. 2.

6. A recombinant vector comprising a polynucleotide according to claim 4 or 5.

7. A host cell comprising a recombinant vector according to claim 6.

8. A carrier on which a polypeptide according to one of the claims 1, 2 or 3 is immobilised.

9. Method for the detection of an infection with pathogens of the genus *Leishmania,* wherein
■ a biological sample of the individual to be examined is brought into contact with a polypeptide according to any one of claims 1 - 3 and
■ the presence of specific antibodies directed to a polypeptide according to any one of claims 1 - 3 in said biological sample is detected, wherein the presence of said specific antibodies in the biological sample is indicative of a *Leishmania* infection.

10. Method according to claim 9, wherein said polypeptide is bound to a carrier.

11. Method according to claim 10, wherein the carrier is a test strip made of a matrix material suitable for immunochromatographic methods.

12. Method according to any one of claims 9 to 11, wherein the test result, i.e. the specific binding of antibodies from the biological sample to said polypeptide, is determined by means of standard values and/or cut off values.

13. Test kit for the immunological detection of an infection with pathogens of the genus *Leishmania,* comprising a carrier according to claim 8.

14. Test kit according to claim 13, wherein a comparison standard for negative values and/or a positive control is additionally available.
